Europäisches Patentamt

(19) European Patent Office

Office européen des brevets

(11) Publication number: **0 001 506**
A1

(12) # EUROPEAN PATENT APPLICATION

(21) Application number: 78300457.5

(22) Date of filing: 05.10.78

(51) Int. Cl.²: **B 01 J 31/22,** C 07 C 121/26, C 07 C 120/00, C 07 C 11/16, C 07 C 3/10

(30) Priority: 11.10.77 GB 42260/77

(43) Date of publication of application: 18.04.79 Bulletin 79/8

(84) Designated Contracting States: **BE DE FR GB NL**

(71) Applicant: **IMPERIAL CHEMICAL INDUSTRIES LIMITED, Imperial Chemical House Millbank, London SW1P 3JF (GB)**

(72) Inventor: **Waddan, Dhafir Yusuf, 35, Linwood Avenue Stokesley, Middlesbrough Cleveland (GB)**

(74) Representative: **Taylor, Michael et al, Imperial Chemical Industries Limited Legal Department: Patents Thames House North Millbank, London SW1P 4QG (GB)**

(54) Process for the production of organic nitriles.

㊗ A $C_5$ mono-olefinically unsaturated mononitrile is disproportionated into a mixture of $C_6$ saturated dinitrile and butadiene by heating with a zerovalent nickel catalyst.

1                                          H.29821|EP.

## Organic Nitriles

THIS INVENTION relates to the manufacture of organic nitriles and more particularly to the conversion of olefinically unsaturated mononitriles to dinitriles.

Our invention provides a process for the disproportionation of a $C_5$ mono-olefinically unsaturated mononitrile into a mixture of $C_6$ saturated dinitrile and butadiene which comprises heating the said mononitrile, in the absence of added hydrogen cyanide, with a zerovalent nickel catalyst.

The $C_5$ mono-olefinically unsaturated mononitrile is preferably one in which the nitrile and olefinic group are unconjugated. Particularly suitable mononitriles are 3-pentenenitrile, 4-pentenenitrile and 2-methyl-3-butenenitrile.

The zerovalent nickel catalyst is preferably of the formula $NiL_4$ where L represents a suitable ligand. Suitable ligands include organic phosphines, arsines, stibines, phosphites, arsenites, stibites, phosphinites and phosphonites. Particularly suitable ligands are those phosphorus compounds of the formula P(XYZ) where X and Y represent R or OR and Z represents OR, where the R's, which may be the same or different, represent alkyl or aryl groups. Preferably the alkyl or aryl groups contain up to 18 carbon atoms. More preferably the alkyl groups contain from 1 to 6 carbon atoms, and the aryl groups are phenyl, tolyl, xylyl or substituted phenyl groups. As examples of such ligands there may be mentioned trimethyl phosphite, triethyl phosphite, triphenyl phosphite, tri(m-tolyl) phosphite, tri-(p-tolyl) phosphite, tri-(p-chloro-phenyl) phosphite and tri-(p-methoxyphenyl) phosphite.

The disproportionation process is effected by heating the said mononitrile with the zerovalent nickel catalyst. The temperature of heating is, for example, within the range 50° to 200°C and is preferably within the range 100° to 150°C.

The disproportionation process is preferably effected in the presence of a Lewis acid. Suitable Lewis acids are, for example, the salts of various metals, especially zinc, aluminium, titanium, tin and iron. The anion of the salt may be inorganic, for example, fluoride, chloride, bromide or iodide, sulphate or phosphate and may be the anion of an organic carboxylic acid, especially a lower aliphatic acid having up to 6 carbon atoms, for example, the acetate.

It may also be advantageous to add ligand in excess of that required to form the zerovalent nickel complex.

If desired the disproportionation process may be carried out in the presence of a solvent. Suitable solvents are, for example, hydrocarbon solvents, e.g. benzene, toluene and xylene, or nitrile solvents, e.g. acetonitrile, propionitrile, benzonitrile and adiponitrile.

The zerovalent nickel catalyst is used in catalytically effective amount, for example in the range of 0.005 to 0.2 moles per mole of mononitrile. The Lewis acid may be used, for example, in the proportion of from 0.5 to 50 moles per mole of catalyst and preferably in the range 1 to 5 moles.

The disproportionation process may be effected, for example, by heating the said mononitrile with the catalyst and optionally Lewis acid, excess ligand and solvent, in a closed vessel under autogeneous pressure. The period of heating may be varied, for example, according to the degree of conversion of starting material desired, and may vary, for example, from 0.5 to 50 hours. At the end of the process the reaction mixture comprises, in addition to
catalyst, unconverted mononitrile and possibly its isomers
formed during the process, butadiene and $C_6$ saturated
dinitriles.  The $C_6$ saturated dinitriles are principally
adiponitrile and 2-methylglutaronitrile.  Any conversion of
the starting mononitrile to an isomer during the process,
for example of 3-pentenenitrile to 4-pentenenitrile or vice
versa is immaterial since the isomers are also converted
to the dinitriles.  The principal components of the
reaction mixture may be separated from each other by any
convenient means, for example by fractional distillation,
desirably in part under reduced pressure.  If desired the
butadiene may alternatively be bled from the reaction
vessel as the process proceeds.

The process of our invention may be used in the
manufacture of adiponitrile from butadiene.  Thus butadiene
may be converted to 3-pentenenitrile by methods known in
themselves, for example by reaction with hydrogen cyanide
in the presence of a copper catalyst as described, for
example, in British Patent Specification No. 1,429,651.
The 3-pentenenitrile may then be disproportionated to
adiponitrile and butadiene according to the process of our
invention and the butadiene recycled for further conversion
to 3-pentenenitrile.

Adiponitrile may be converted by hydrogenation
to hexamethylene diamine a valuable intermediate for the
manufacture of polyamides and polyurethanes, especially of
polyhexamethylene adipamide (nylon 6,6) useful in the
manufacture of fibres, films and mouldings.

The invention is illustrated but not limited by
the following Examples in which the parts and percentages
are by weight.
EXAMPLES 1 to 4

3-pentenenitrile (5 parts), nickel tetrakis-
(tri-p-tolylphosphite) (0.5 part) and anhydrous zinc
chloride (0.2 part, 2 moles/mole of catalyst) were heated
at 120°C for 9 hours.  After cooling, butadiene was
removed by evaporation and the residue was found to contain 1.2% of adiponitrile and 2.4% of glutaronitrile by gas/liquid chromatography.

The experiment was repeated under varied conditions which are set out in the following table together with the results.

TABLE

| Example No. | Catalyst | Molar Ratio ZnCl$_2$/Catalyst | Other Variants | ADN % | 2MGN % |
|---|---|---|---|---|---|
| 1 | II | 2 : 1 | - | 1.2 | 2.4 |
| 2 | II | 1 : 1 | - | 1.1 | 2.8 |
| 3 | I | 4 : 1 | + 1 mole excess ligand per mole catalyst | 1.75 | 1.4 |
| 4 | II | 2 : 1 | + 1 mole excess ligand per mole catalyst | 4.04 | 5.54 |

I = nickel tetrakis (tri-ethyl phosphite)

II = nickel tetrakis (tri-p-tolyl phosphite)

ADN = adiponitrile

2MGN = 2-methylglutaronitrile

EXAMPLE 5

4-pentenenitrile (5 parts), nickel tetrakis-(tri-p-tolylphosphite) (0.5 part) and anhydrous zinc chloride (0.1 part) were heated at 120°C for 18 hours. After cooling and evaporating butadiene the residue was found to contain 4.3% of adiponitrile and 3.8% of 2-methylglutaronitrile.

Claims

1. A process for the disproportionation of a $C_5$ mono-olefinically unsaturated mononitrile into a mixture of $C_6$ saturated dinitrile and butadiene characterised in that the said mono-nitrile is heated, in the absence of added hydrogen cyanide, with a zerovalent nickel catalyst.

2. A process according to claim 1 characterised in that the mononitrile is 3-pentenenitrile, 4-pentenenitrile or 2-methyl-3-butenenitrile.

3. A process according to claim 1 or claim 2 characterised in that the zerovalent nickel catalyst has the formula $NiL_4$ where L represents a ligand which is an organic phosphine, arsine, stibine, phosphite, arsenite, stibite, phosphinite or phosphonite.

4. A process according to claim 3 characterised in that the liquid is a phosphorus compound of formula P(XYZ) where X and Y represent R or OR and Z represents OR, where the R's, which may be the same or different, represent alkyl or aryl groups.

5. A process according to claim 4 characterised in that the alkyl groups contain from 1 to 6 carbon atoms and the aryl groups are phenyl, tolyl, xylyl or substituted phenyl groups.

6. A process according to claim 5 characterised in that one or more of the ligands is trimethyl phosphite, triethylphosphite, triphenyl phosphite, tri-(m-tolyl) phosphite, tri-(p-tolyl) phosphite, tri-(p-chlorophenyl) phosphite or tri-(p-methoxyphenyl) phosphite.

7. A process according to any one of the preceding claims characterised in that the temperature is 50° to 200°C.

8. A process according to any one of the preceding claims characterised in that a Lewis acid is present.

2

0001506

9. A process according to claim 8 characterised in that the Lewis acid is a fluoride, chloride, bromide, iodide, sulphate, phosphate or organic carboxylate of zinc, aluminium, titanium, tin or iron.

10. A process according to any one of the preceding claims characterised in that a solvent is present.

11. A process according to claim 10 characterised in that the solvent is a hydrocarbon or a nitrile.

12. A process for the preparation of adiponitrile which comprises converting butadiene to 3-pentenenitrile by a known method and disproportionating the 3-pentenenitrile to adiponitrile by a process according to any one of the preceding claims.

## DOCUMENTS CONSIDERED TO BE RELEVANT

| Category | Citation of document with indication, where appropriate, of relevant passages | Relevant to claim |
|---|---|---|
| A | <u>US - A - 3 852 329</u> (TOMLINSON) <br> * Claims 1-12 * <br><br> -- | 1 |
| A | <u>US - A - 3 852 328</u> (YUAN-TSAN CHIA) <br> * Claims 1-8 * <br><br> -- | 1 |
| A | <u>US - A - 3 846 474</u> (Y.I. MOK) <br> * Claims 1-18 * <br><br> ---- | 1 |

**CLASSIFICATION OF THE APPLICATION (Int. Cl.²)**

B 01 J  31/22
C 07 C 121/26
        120/00
        11/16
        3/10

**TECHNICAL FIELDS SEARCHED (Int.Cl.²)**

C 07 C 121/26
        120/00
B 01 J  31/22

**CATEGORY OF CITED DOCUMENTS**

X: particularly relevant
A: technological background
O: non-written disclosure
P: intermediate document
T: theory or principle underlying the invention
E: conflicting application
D: document cited in the application
L: citation for other reasons
&: member of the same patent family, corresponding document

The present search report has been drawn up for all claims

| Place of search | Date of completion of the search | Examiner |
|---|---|---|
| The Hague | 18-12-1978 | DE ROY |